Europäisches Patentamt

(19) European Patent Office

Office européen des brevets

(11) Publication number: **0 288 311**
**A1**

## (12) EUROPEAN PATENT APPLICATION

(21) Application number: **88303665.9**

(22) Date of filing: **22.04.88**

(51) Int. Cl.⁴: **G 01 N 33/76**

(30) Priority: **24.04.87 US 43020**

(43) Date of publication of application:
**26.10.88 Bulletin 88/43**

(84) Designated Contracting States: **DE FR IT**

(71) Applicant: **PALL CORPORATION**
**30 Sea Cliff Avenue**
**Glen Cove New York 11542 (US)**

(72) Inventor: **Pascale, Frank R.**
**12 Francis Court**
**Glen Cove New York 11542 (US)**

(74) Representative: **Corin, Christopher John et al**
**Mathisen Macara & Co. The Coach House 6-8 Swakeleys Road**
**Ickenham Uxbridge UB10 8BZ (GB)**

(54) Method of treating urine.

(57) A microfibrous web is used to rectify a urine sample, and thereby enhance its subsequent analysis. The method comprises contacting the urine sample with a microfibrous web of synthetic polymeric microfibers and, in a preferred embodiment, thereafter contacting the rectified urine with a microporous reaction membrane. Preferred microfibrous webs are comprised of polypropylene microfibers having diameters in the range of from 1 to 20 micrometers.

EP 0 288 311 A1

Bundesdruckerei Berlin

## Description

## METHOD OF TREATING URINE

This invention relates to a method of treating urine. More particularly, this invention is directed to a method for rectifying urine samples to enhance the accuracy of a subsequent analysis.

Methods for determining the presence of an analyte in bodily fluids often rely on the use of a microporous membrane which functions as a substrate for biochemical reactions. In such methods, the bodily liquid must initially pass through the membrane in sufficient quantity to assure that the specific analyte, if present, can react with a component already present on the membrane, for example, a ligand bound to the surface of the membrane. In subsequent steps, other liquids are passed through the membrane, culminating in an observable phenomenon, e.g., a color development, if the specific analyte was originally present. If a sufficient quantity of the bodily fluid, or any of the subsequent liquids, cannot pass through the membrane, the test results may be inaccurate or even impossible to obtain.

In general, reduction or stoppage of liquid flow through the membrane almost always occurs during the addition of the bodily liquid. Since these liquids are often complex biological substances, they may contain components that can block the fine pores of the membrane which typically range from 0.2 to 5 microns in diameter. Moreover, many of these liquids may exhibit variable flow behavior depending on diverse factors, including the disease state of the patient supplying the sample, or even the time of day when the liquid was obtained.

Urine is a classic example of a bodily fluid which is known to exhibit variable flow behavior in test kits employing microporous membranes. Typically, in about 10 seconds, several hundred microliters (about 6 drops) of an ordinary urine sample can flow through a membrane which has a pore rating of 3 micrometers and a surface area of 3 cm². The same amount of urine, taken as the first sample of the morning, may take up to or over 90 seconds to flow through the membrane, or the urine sample may entirely block the membrane after the first 2 or 3 drops. This effect is most often seen in pregnancy tests which use morning urine. Morning urine is preferred for pregnancy tests because it contains the greatest concentration of the analyte. Conversely, however, it is also the most susceptible to blockage because of the greater concentration of deleterious components.

The subject invention is directed to a method for treating urine which substantially overcomes the types of problems discussed above, thereby providing more accurate test results.

In accordance with this invention, a method is provided for rectifying urine, and thereby enhancing its subsequent analysis. The method comprises contacting the urine with a microfibrous web of synthetic polymeric microfibers and, in a preferred embodiment, thereafter contacting the rectified urine with a microporous reaction membrane.

Preferred microfibrous webs are comprised of polypropylene microfibers having diameters in the range of from 1 to 20 micrometers.

As summarized above, in accordance with the subject invention, a method is provided for treating urine which substantially overcomes the types of problems discussed above.

To illustrate an application in accordance with the subject invention, a sample of morning urine from a pregnant woman, shown to completely block a first sample of a microporous reaction membrane, can be rectified by contacting the urine sample with a microfibrous web of synthetic polymeric microfibers, following which the urine sample passes freely through a second sample of the same microporous membrane.

Useful microfibrous webs include melt-blown or spun-bonded microfibrous webs of thermoplastic polymers such as polyolefins (such as polypropylene and polyethylene), polyesters (such as polyethylene terephthalate and polybutylene terephthalate), polyamides (such as nylon 66), and the like. Particularly preferred is polypropylene. These microfibrous webs are characterised by being formed of entangled, non-woven microfibers which are typically in the range of from 1 to 50 micrometers. Microfibrous webs prepared from microfibers of two or more different polymers may also be used. These structures typically have filtration removal ratings in liquid service, ranging from 0.2 to 100 micrometers for particulates. Microfibrous web weights typically vary from 1.08 to 530 grams/square meter (0.1 to 50 grams per square foot), with thicknesses of from 0.013 to 1.27 centimeters (0.005 to 0.50 inches). Void volumes typically range from 30 to 90%.

Preferably, the urine sample is contacted with the layer of microfibrous web in one of two ways. The sample may be passed through the microfibrous web unidirectionally, or it may be permitted to flow through a layer of the microfibrous web bidirectionally, i.e., through the web in one direction and then back through the web in the opposite direction. In this latter approach, a preferred device takes the form of a hollow elongated body having a rubber bulb at one end and a microporous web secured across the opposite open end. When the end of the device with the microfibrous web secured across it is inserted into a container of urine and the depressed rubber bulb on the opposite end is released, urine is pulled into the hollow body (as a result of the vacuum created therein) and the urine contacts the microfibrous web as it enters. Thereafter, the device can be removed from the container of urine, and the bulb again depressed to expel the urine from the hollow body, e.g., onto a microfibrous reaction membrane. In such a manner, the urine is bidirectionally contacted with the microfibrous web and is rectified prior to contact with the reaction membrane. Alternatively, but less desirably, the urine sample can be contacted with the web by placing the sample in a container together with the microfibrous web and agitating the urine sample while in the presence of the microfibrous web to achieve good contact.

The microfibrous web is comprised of one or more synthetic polymers such as those materials described above, but is most preferably polypropylene. The microfibrous web weights of the material will preferably be in the range of from 5.38 to 323 grams/square meter (0.5 to 30 grams per square foot), more preferably in the range of from 10.8 to 269 grams/square meter (1 to 25 grams per square foot), and will typically have thicknesses in the range of from 0.013 to 1.27 centimeters (0.005 to 0.50 inches), preferably from 0.018 to 0.64 centimeters (0.007 to 0.250 inches), and most preferably from 0.025 to 0.38 centimeters (0.010 to 0.150 inches). Voids volumes in the webs are typically in the range of from 30 to 90%, more preferably from 50 to 80%. The selection of the material and the weight per square foot will depend on the nature of the analyte and the nature of the deleterious component(s). For example, if the analyte is a protein, the smallest amount of the microfibrous web which removes the blocking substance is preferable, since some protein will also be removed by contact with the web. An analyte which is bacterial will tolerate a larger amount of microfibrous web. Choices of other polymers for the microfibrous web may be tailored to the specific nature of the analytes and the blocking substances.

As described above, the use of a microfibrous web in a pretreatment step can rectify urine samples which might otherwise block and render inaccurate or unusable the microporous membrane used as a substrate or reaction surface in diagnostic test kits. A variety of devices may be used to provide the requisite contact.

The invention will be better understood by reference to the following example which is offered by way of illustration.

Example

In an actual test, a sample of morning urine was placed onto the upper surface of an assay device consisting of a 3 micrometer pore rated, nylon 66 membrane having a face surface area of about 133 mm$^2$ and whose lower surface was in contact with an absorbent pad. In this test, a total of 6 drops of urine was applied to the upper surface of the membrane, 3 drops at a time. The flow time for absorption of the first 3 drops, i.e., the disappearance from the upper surface, was 7 seconds. The flow time for the disappearance of the second 3 drops was greater than 60 seconds.

Another aliquot of the same urine sample was drawn up through a device as described above, having an elongated body, a rubber bulb at one end, and a microfibrous web of polypropylene positioned at the opposite end. This device provided bidirectional contact of the urine with the web which had a web weight of 32 grams per square meter (3 grams per square foot), a face surface area of about 79 mm$^2$ and a thickness of 0.05 centimeters (0.020 inches). The web was comprised of microfibers having diameters of about 2 micrometers. In this test, the urine sample was discharged from the device, 3 drops at a time, onto a second assay device of the same design as described above. The flow time for absorption of the first 3 drops was 3 seconds. The flow time for the second 3 drops was also 3 seconds. An additional 6 drops of conjugate solution, necessary for subsequent biochemical reactions, was then applied to the assay device. The flow time for all 6 drops of the conjugate solution was 8 seconds.

The prompt flow times for this test sequence indicates that the membrane in the assay device was rendered free of those obstructing substances in the urine sample which effectively blocked the membrane in the initial test.

The subject invention has particular application in the analysis of urine for pregnancy determination. In such a test, the rectified urine is applied to the surface of a microporous membrane which has been pretreated with an antibody which specifically binds HCG (human chorionic gonadotropin) hormone. HCG appears in the urine several days after conception. After application of the urine to the membrane, a wash buffer is applied to the membrane to rinse out unbound material. HCG, if present, will be retained within the membrane. A sequence of additional fluids, including detection reagents and color developers, are then added. If a positive result is obtained, it will be evidenced by a change in color.

Claims

1. A method of rectifying urine comprising contacting the urine sample with a microfibrous web of synthetic polymeric microfibers.

2. The method of claim 1, wherein the polymeric microfibers are comprised of a polymer selected from the group consisting of polyolefins, polyesters, polyamides, and mixtures thereof.

3. The method of claim 2, wherein said microfibrous web is comprised of microfibers having diameters in the range of from 1 to 50 micrometers, said microfibrous web has a web weight of 1.08 to 538 grams/square meter (0.1 to 50 grams per square foot) and a voids volume of from 30 to 90%.

4. The method of claim 3, wherein said polymeric microfibers are comprised of polypropylene, said microfibrous web has a microfibrous web weight of from 5.38 to 323 grams/ square meter (0.5 to 30 grams per square foot) and a voids volume of from 50 to 80%.

5. The method of claim 1, wherein said urine sample is contacted with said web by passing said urine sample through said web.

6. A method of carrying out an analysis of a urine sample comprising:

(1) contacting the urine sample with a microfibrous web of synthetic polymeric microfibers to rectify the sample, and

(2) contacting the rectified urine sample with a microporous reaction membrane.

7. The method of claim 6, wherein the polymeric microfibers are comprised of a polymer selected from the group consisting of polyolefins, polyesters, polyamides, and mixtures thereof.

8. The method of claim 7, wherein said microfibrous web is comprised of microfibers having diameters in the range of from 1 to 50 micrometers, said microfibrous web has a web weight of from 1.08 to 538 grams/square meter (0.1 to 50 grams per square foot) and a voids volume of from 30 to 90%.

9. The method of claim 8, wherein said polymeric microfibers are comprised of polypropylene, said microfibrous web has a microfibrous web weight of from 5.38 to 323 grams/ square meter (0.5 to 30 grams per square foot) and a voids volume of from 50 to 80%.

10. The method of claim 6, wherein said urine sample is contacted with said web by passing said urine sample through said web.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-2 951 672 (TAKEDA CHEMICAL INDUSTRIES) * page 7, lines 23-30; page 8, lines 18-24; page 11, lines 8-33; tables 4,5 * | 1,2,5,7,10 | G 01 N 33/76 |
| | --- | | |
| X | DE-A-2 208 269 (MOCHIDA SEIYAKU K.K.) * claim 1 * | 1,5,10 | |
| Y | | 6 | |
| | --- | | |
| Y | US-A-4 123 509 (U.K. BANIK et al.) * claim 1 * ----- | 6 | |

TECHNICAL FIELDS SEARCHED (Int. Cl.4)

G 01 N 33/76
G 01 N 33/543
G 01 N 33/545
B 01 D 13/00

The present search report has been drawn up for all claims

| Place of search | Date of completion of the search | Examiner |
|---|---|---|
| BERLIN | 23-06-1988 | CORDERO ALVAREZ M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)